# EUROPEAN PATENT APPLICATION

(11) **EP 1 344 541 A2**
(43) Date of publication of application: **17.09.2003**
(21) Application number: 03005687.3
(22) Date of filing: 13.03.2003
(51) Int. Cl.: A61M 1/10, F04D 29/24

(54) **Centrifugal pump**

(30) Priority: 14.03.2002 JP 2002070853
(71) Applicant: SUN MEDICAL TECHNOLOGY RESEARCH CORPORATION, Suwa-shi, Nagano (JP)
(72) Inventor: Tajima, Koki, Suwa-shi, Nagano (JP); Kitano, Tomoya, Suwa-shi, Nagano (JP)
(74) Representative: Leson, Thomas Johannes Alois, Dipl.-Ing.

(57) **Abstract**

A centrifugal pump prevents a fluid from stagnating and so stops decreases in pump efficiency. An open impeller-type centrifugal pump includes pump vanes 12 that are connected via a vane boss 10 to an end part of a rotational shaft 8, a casing 14 that is connected to an end part of the pump base part 6 and forms a fluid chamber R surrounding the pump vanes 12, and an intake 16 and an outtake 18 that are formed in the casing 14. The pump vanes 12 are composed of pump vanes 12a to 12c that are integrally formed with current plates 20a to 20c. The current plates 20a to 20c cause some of the fluid that enters via the intake 16 to flow downwards along the axial direction of the rotational shaft 8. The fluid that flows along the current plates causes a centrifugal flow from an inner periphery to an outer periphery in a gap between a pump base part and the lower surfaces of the pump vanes.

## Description

### BACKGROUND OF THE INVENTION

### 1. Field of the Invention

The present invention relates to a centrifugal pump that suppresses the stagnation of fluid inside the fluid chamber.

### 2. Background Art

The centrifugal pump disclosed in Japanese Laid-Open Patent Application No. 10-015056 (hereinafter referred to as "background art example 1") and the centrifugal pump disclosed in Japanese Laid-Open Patent Application No. 09-206372 (hereinafter referred to as "background art example 2") are two examples of conventional centrifugal pumps.

In the centrifugal pump of background art example 1, a rotational shaft that extends from an end part of the driving unit is connected to a vane boss of the pump vanes. The end part of the driving unit is connected to the casing that covers the pump vanes so as to form a fluid chamber. An intake, which guides fluid into the fluid chamber, is provided on the casing facing the axial direction of the rotation shaft and an outtake, from which fluid that has been accelerated by the centrifugal force generated by the pump vanes is expelled, is provided on the side of the casing, making the background art example 1 an open impeller-type centrifugal pump. Rotation of the pump vanes generates a flow of fluid from the intake towards the outtake, so that fluid is pumped from the intake to the outtake at a predetermined flow rate.

It should be noted that in this specification, the term "a pump vane" means "a pump impeller" or "a pump impeller blade" depending on the situation.

The centrifugal pump of the background art example 2 is a closed impeller-type centrifugal pump including an impeller where a plurality of partition plates are disposed between a pair of disc-shaped magnetic members, a housing that forms a fluid chamber by surrounding the impeller and is internally equipped with a support part for supporting the impeller so that the impeller is rotatable, an intake that is provided at a predetermined position on the housing so as to face a passage provided at a rotational center position of the impeller, an outtake that is provided at a predetermined position on the housing so as to face the impeller at an outer part in a radial direction, and an impeller rotational torque generating unit composed of a rotor magnet in a lower part in the housing. When the impeller is rotated by the action of the impeller rotational torque generating unit, a centrifugal force is applied to the fluid that has been introduced into the impeller from the intake, so that fluid is pumped from the intake to the outtake at a predetermined flow rate.

However, with the centrifugal pump of background art example 1, some of the high-pressure fluid that attempts to flow into the outtake flows back towards the low-pressure fluid in a gap between the end part of the driving unit and the lower surfaces of the pump vanes. In some cases, fluid stagnates in this gap, which can lower the pump's efficiency. When the centrifugal pump is used as a blood pump for pumping blood, the stagnant blood in the gap between an end part of the driving unit and the lower surfaces of the pump vanes coagulates, which can result in the formation of a thrombus.

The centrifugal pump of background art example 2 is the same in that there are cases were some high-pressure fluid at the outtake flows back towards the low-pressure fluid in a gap between the housing and the lower surfaces of the pump vanes. This causes fluid to stagnate in this gap and can lower the pump's efficiency. When the centrifugal pump is used as a blood pump for pumping blood, the stagnant blood (or pooled blood) can coagulate, which may result in the formation of a thrombus.

### SUMMARY OF THE INVENTION

The present invention was conceived in view of the above situation and has an object of providing a centrifugal pump that prevents the stagnation of fluid and so prevents decreases in pump efficiency and additionally prevents the formation of a thrombus when used as a blood pump.

According to a first aspect of the present invention, a centrifugal pump includes a rotational shaft that is disposed on a pump base part, at least one pump vane that is radially connected via a vane boss to an end part of the rotational shaft, a casing that is connected to the pump base part and forms a fluid chamber surrounding the at least one pump vane, a fluid intake (a fluid suction opening) that is provided at a predetermined position on the casing, and a fluid outtake (a fluid discharge opening) that is provided at a predetermined position on the casing, the centrifugal pump further including a stagnation preventing unit for generating, in a gap between the pump base part and lower surfaces of the at least one pump vane, a fluid flow in a centrifugal direction that is directed towards the fluid outtake.

According to a second aspect of the present embodiment, in the centrifugal pump of the first aspect, the stagnation preventing unit may be at least one current plate that is provided on the vane boss, the at least one current plate causing some of a fluid that enters via the fluid intake to flow in an axial direction towards the pump base part, causing acceleration in the fluid flowing in the axial direction, and making the fluid flow towards the gap between the pump base part and the lower surfaces of the at least one pump vane.

According to a third aspect of the present invention, in the centrifugal pump of the second aspect, each of the at least one current plate may be integrally formed with base parts of the at least one pump vane and may be connected to the vane boss.

According to a fourth aspect of the present invention, in the centrifugal pump of the second aspect, each of the at least one current plate may be a wing-shaped member that is connected to the vane boss at a different position to base parts of the at least one pump vane.

With the centrifugal pumps of the first to fourth aspects of the present invention, the stagnation preventing unit generates a fluid flow in the centrifugal direction towards the fluid outtake in a gap between the pump base part and the lower surfaces of the pump vanes, so that fluid does not stagnate in the gap and the pump efficiency can be raised.

With the centrifugal pumps of the second to fourth aspects of the present invention, the at least one current plate that forms the stagnation preventing unit causes some of a fluid that enters via the fluid intake to flow in an axial direction towards the pump base part, causes acceleration in the fluid flowing in the axial direction, and makes the fluid flow towards the gap between the pump base part and the lower surfaces of the at least one pump vane, so that the stagnation of fluid can be reliably prevented.

According to a fifth aspect of the present invention, in the centrifugal pump of any of the first to fourth aspects, a plurality of current channels (or groove), which extend radially from the rotational shaft and are bent on the radial direction, may be formed in at least one of a surface of the pump base part, the lower surfaces of the at least one pump vane, and lower surfaces of the at least one current plate.

With the centrifugal pump of the fifth aspect of the present invention, a plurality of current channels, which extend radially from the rotational shaft and are bent on the radial direction, may be formed in a surface of the pump base part that faces the at least one pump vane. This increases the flow in the centrifugal direction in the gap and makes it possible to prevent the stagnation of fluid even more thoroughly.

According to a sixth aspect of the present invention, a centrifugal pump includes a pump vane in which a plurality of blades are disposed radially on a ring shaped first shroud, a casing that is connected to a pump base part and forms a fluid chamber surrounding the pump vane, a fluid intake that is provided at a predetermined position on the casing, and a fluid outtake that is provided at a predetermined position on the casing, the centrifugal pump further including a stagnation preventing unit for generating, in a gap between a lower surface of the pump vane and the pump base part, a flow in a centrifugal direction that is directed towards the fluid outtake.

According to a seventh aspect of the present invention, a centrifugal pump includes a pump vane where a plurality of blades are disposed radially on a ring-shaped first shroud and a disc-shaped second shroud with a central fluid passage hole is disposed on the blades, a casing that is connected to a pump base part and forms a fluid chamber surrounding the pump vane, a fluid intake that is provided at a predetermined position on the casing, and a fluid outtake that is provided at a predetermined position on the casing, the centrifugal pump further including a stagnation preventing unit for generating, in a gap between a lower surface of the pump vane and the pump base part, a flow in a centrifugal direction that is directed towards the fluid outtake.

According to an eighth aspect of the present invention, in the centrifugal pump of any of the sixth and seventh aspects, the stagnation preventing unit may be at least one current plate that is disposed on the lower surface of the pump vane facing the pump base part, the at least one current plate agitating fluid that stagnates in the gap between the lower surfaces of the pump vane and the pump base part and generating the flow in the centrifugal direction towards the fluid outtake.

According to a ninth aspect of the present invention, in the centrifugal pump of any of the seventh and eighth aspects, a plurality of current channels, which extend radially from a center of rotation of the pump vane and are bent on the radial direction, may be formed in at least one of a lower surface of the first shroud, a lower surface of the pump vane, and lower surfaces of the at least one current plate.

With the centrifugal pump of the sixth to ninth aspects of the present invention, the stagnation preventing unit generates, in the gap between the pump base part and the lower surface of the pump vane, a centrifugal flow of fluid towards the fluid outtake, so that fluid does not stagnate in the gap and the pump efficiency can be raised.

With the centrifugal pump of the eighth aspect of the present invention, the at least one current plate that composes the stagnation preventing unit agitates fluid that stagnates in the gap between the lower surfaces of the pump vane and the pump base part and so generates a flow in the centrifugal direction towards the fluid outtake. In this way, the stagnation of fluid can be reliably prevented.

With the centrifugal pump of the ninth aspect of the present invention, a plurality of current channels, which extend radially from a center of rotation of the pump vane and are bent on the radial direction, are formed in at least one of the lower surface of the first shroud and the surface of the pump base part. This increases the flow in the centrifugal direction, which makes it possible to further prevent the stagnation of fluid.

According to a tenth aspect of the present invention, in the centrifugal pump of any of the first to ninth aspects, the fluid may be blood.

With the centrifugal pump of the tenth aspect of the present invention, the centrifugal pump can be used as a blood pump that reliably prevents the formation of a thrombus.

### BRIEF DESCRIPTION OF THE DRAWINGS

FIG. 1 shows a centrifugal pump according to a first embodiment of the present invention.
FIG. 2 shows pump vanes that compose the centrifugal pump according to the first embodiment.
FIG. 3 shows a surface of a pump base part that faces the pump vanes in the centrifugal pump according to the first embodiment.
FIG. 4 is a cross-sectional view showing a modification of current plates that serve as a stagnation preventing means and are integrated with the pump vanes that compose the centrifugal pump of the first embodiment.
FIG. 5 shows another modification of the current plates that serve as the stagnation preventing means and are integrated with the pump vanes that compose the centrifugal pump of the first embodiment.
FIG. 6 shows a third embodiment directed to current plates that serve as the stagnation preventing means and have a separate construction to the pump vanes.
FIG. 7 shows the centrifugal pump according to a fourth embodiment of the present invention.
FIG. 8 shows the pump vanes that compose the centrifugal pump according to the fourth embodiment.
FIG. 9 shows the current plates that are integrated with the blades that compose the pump vane shown in FIG. 8.
FIG. 10 shows another embodiment directed to the current plates that are integrated with the blades in the pump vane.
FIG. 11 shows yet another embodiment directed to the current plates that are integrated with the blades in the pump vane.
FIG. 12 shows yet another embodiment directed to the current plates that are integrated with the blades in the pump vane.
FIG. 13 shows yet another embodiment directed to the current plates that are integrated with the blades in the pump vane.
FIG. 14 shows yet another embodiment directed to the current plates that are integrated with the blades in the pump vane.
FIG. 15 shows yet another embodiment directed to the current plates that are integrated with the blades in the pump vane.
FIG. 16 shows a fifth embodiment that is directed to a pump vane of a different construction.
FIG. 17 shows current plates with a different construction to the current plates of the pump vane shown in FIG. 16.
FIG. 18 shows current plates with a different construction to the current plates of the pump vane shown in FIG. 17.
FIG. 19 shows the pump vane according to a sixth embodiment of the present invention from the rear side.
FIG. 20 shows a pump vane according to a seventh embodiment of the present invention from the rear side.
FIG. 21 shows a pump vane according to an eighth embodiment of the present invention.

### DESCRIPTION OF THE PREFERRED EMBODIMENTS

The following describes, with reference to the enclosed drawings, several embodiments of a centrifugal pump according to the present invention.

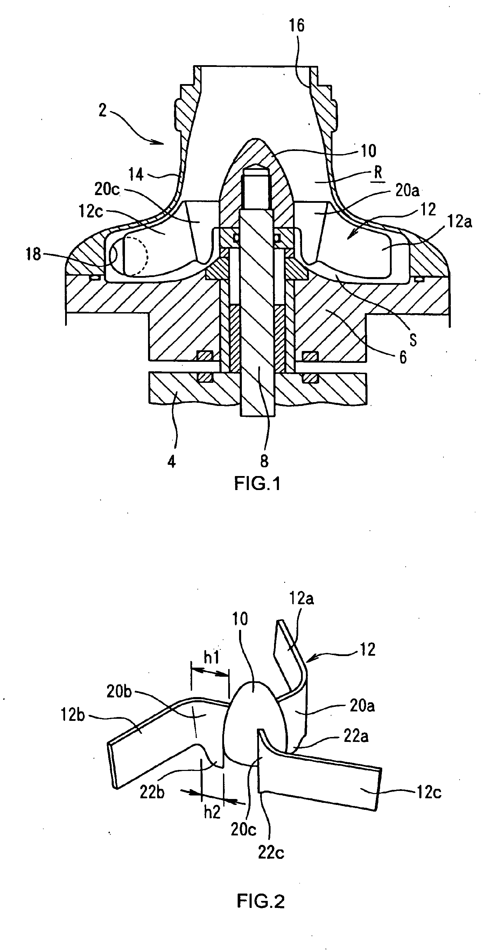

FIGS. 1 and 2 show a centrifugal pump according to a first embodiment that is used as a blood pump. It should be noted that this first embodiment corresponds to Claims 1 to 3 of the present invention.

The centrifugal pump 2 of the present embodiment is an open impeller-type centrifugal pump including a rotational shaft 8 that passes through a pump base part 6 and is connected to a rotor (not shown in the drawing) enclosed in a driving unit 4, pump vanes 12 that are connected, via a vane boss 10, to an end part of the rotational shaft 8 radially in directions that are perpendicular to the rotational shaft 8, a casing 14 that is connected to an end part of the pump base part 6 and forms a fluid chamber R surrounding the pump vanes 12, an intake 16 that is provided in an upper part of the casing 14 facing the end of the vane boss 12, and an outtake 18 that is provided in a side part of the casing 14 facing the pump vanes 12 from outside the pump vanes 12 in the radial direction.

As shown in FIG. 2, the pump vanes 12 are composed of three pump vanes 12a to 12c that are connected to the outer circumference of the vane boss 10 at intervals of 120° around the axis. Each of these pump vanes 12a to 12c is integrally provided with a current plate 20a to 20c that corresponds to a stagnation preventing means of the present invention. In more detail, the current plates 20a to 20c are formed between the base part of each of the pump vanes 12a to 12c and the vane boss 10, and form projecting parts 22a to 22c that extend downwards at an angle that is slightly skewed with respect to the axis of the vane boss 10. The current plates 20a to 20c are approximately trapezoidal in shape with the plate width h1 at the tip end of the vane boss 10 being larger than the plate width h2 of the projecting parts 22a to 22c (that is, h1>h2).

In the centrifugal pump 2 of the above construction, when the rotational shaft 8 rotates due to the operation of the driving unit 4, the pump vanes 12 that rotate in a predetermined direction produce a fluid flow of blood, which has entered the fluid chamber R from the intake 16, towards the outtake 18, thereby pumping a predetermined amount of blood from the intake 16 to the outtake 18.

At this point, the current plates 20a to 20c formed between the vane boss 10 and the base parts of the pump vanes 12a to 12c direct some of the blood that has entered via the intake 16 to generate a downward flow along the axial direction of the rotational shaft 8. The current plates 20a to 20c are formed so that the width of the plates decreases gradually from the side on which the tip end of the vane boss 10 is located, so that the blood that flows along the current plates 20a to 20c accelerates as it flows downwards. This accelerating blood flowing downwards becomes a centrifugal flow from an inner periphery towards an outer periphery in a gap S between the pump base part 6 and the lower surfaces of the pump vanes 12a to 12c.

Even if high-pressure fluid that attempts to flow into the outtake 18 flows back towards the low-pressure blood in the gap S and blood attempts to stagnate in this gap S, the blood from the intake 16 that is directed by the current plates 20a to 20c generates a centrifugal flow from the inner periphery towards the outer periphery in the gap S between the pump base part 6 and the lower surfaces of the pump vanes 12a to 12c, so that blood does not stagnate in the gap S. This reliably prevents a thrombus being formed. Since no blood stagnates, the centrifugal pump 2 does not suffer from a decrease in pump efficiency and so has superior pump performance.

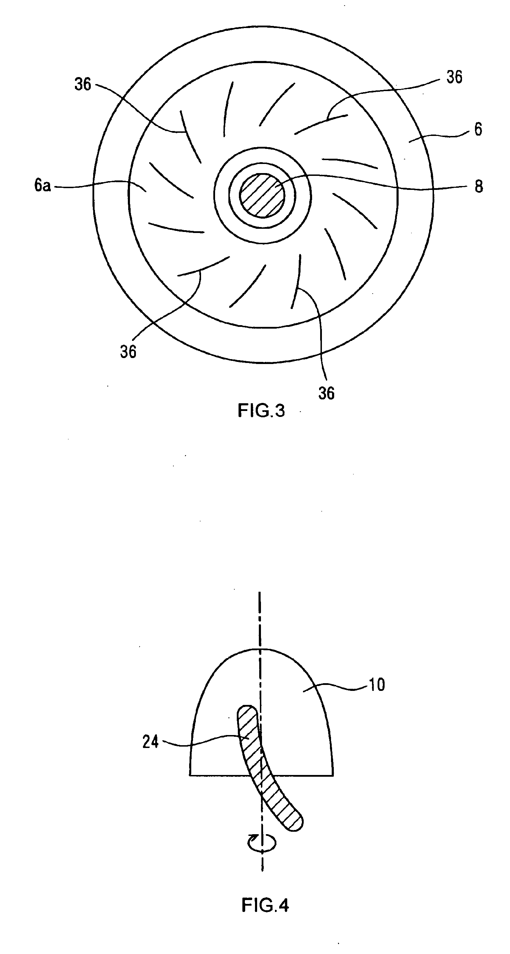

FIG. 3 shows a second embodiment directed to the pump base part 6 described above. It should be noted that this embodiment corresponds to Claims 5 and 9.

The pump base part 6 of this second embodiment has a plurality of current channels 36, which extend radially from the rotational shaft 8 and are bent on the radial direction, in a surface 6a that faces the lower surfaces of the pump vanes 12. The depth of these current channels 36 is set at around 0.1 to 1mm.

By providing the current channels 36 of the above form in the surface 6a of the pump base part 6, a centrifugal flow from the inner periphery to the outer periphery is generated in the gap S between the pump base part 6 and the lower surfaces of the pump vanes 12. By additionally generating this kind of centrifugal flow, the flow in the centrifugal direction is increased, so that the stagnation of blood in the gap S can be avoided and the formation of a thrombus can be prevented even more reliably.

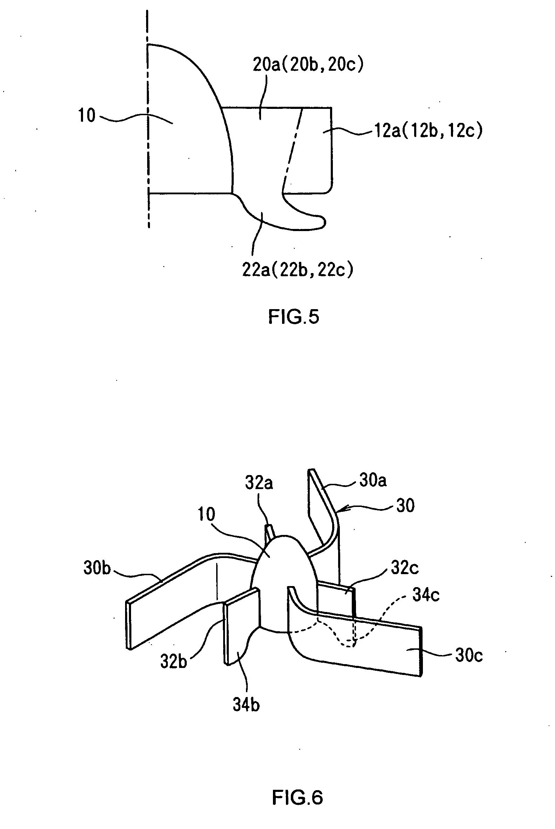

By forming the current plates 20a to 20c so that they are shaped like wings in cross-section in the axial direction as shown in FIG. 4 and/or forming projecting parts 22a to 22c that extend on the radial direction at the bottom ends of the current plates 20a to 20c as shown in FIG. 5, the speed of the flow of blood in the centrifugal direction in the gap S between the pump base part 6 and the lower surfaces of the pump vanes 12 is further increased, so that the blood that stagnates in the gap S can be efficiently pumped to the outtake 18.

FIG. 6 shows a third embodiment directed to the pump vanes that are used in an open-impeller-type centrifugal pump. It should be noted that this third embodiment corresponds to Claim 4.

The pump vanes 30 of the present embodiment are composed of three pump vanes 30a to 30c, which are connected to the outer circumference of the vane boss 10 at intervals of 120° around the axis of the vane boss 10, and three current plates 32a to 32c, which are connected to the outer circumference of the vane boss 10 in the intervals between these pump vanes 30a to 30c. The current plates 32a to 32c are formed with projecting parts 34a to 34c (projecting part 34a is not shown in the drawing) that extend downwards at an angle that is slightly skewed with respect to the axis of the vane boss 10, with the width of the current plates 32a to 32c at the tip end of the vane boss 10 being larger than the width on the same end as the projecting parts 34a to 34c.

In the centrifugal pump 2 that includes the pump vanes 30 of the above construction, even if blood attempts to stagnate in the gap S between the pump base part 6 and the lower surfaces of the pump vanes 30a to 30c, some of the blood from the intake 16 is directed by the current plates 32a to 32c and so creates a centrifugal flow from the inner periphery towards the outer periphery in the gap S between the pump base part 6 and the lower surfaces of the pump vanes 30a to 30c. This means that blood does not stagnate in the gap S, thereby reliably preventing a thrombus from being formed. In addition, the centrifugal pump does not suffer from a decrease in pump efficiency.

It should be noted that while the open-impeller-type centrifugal pump 2 described above is composed of three pump vanes 12a to 12c (30a to 30c), the present invention does not place limitations on the number of pump vanes.

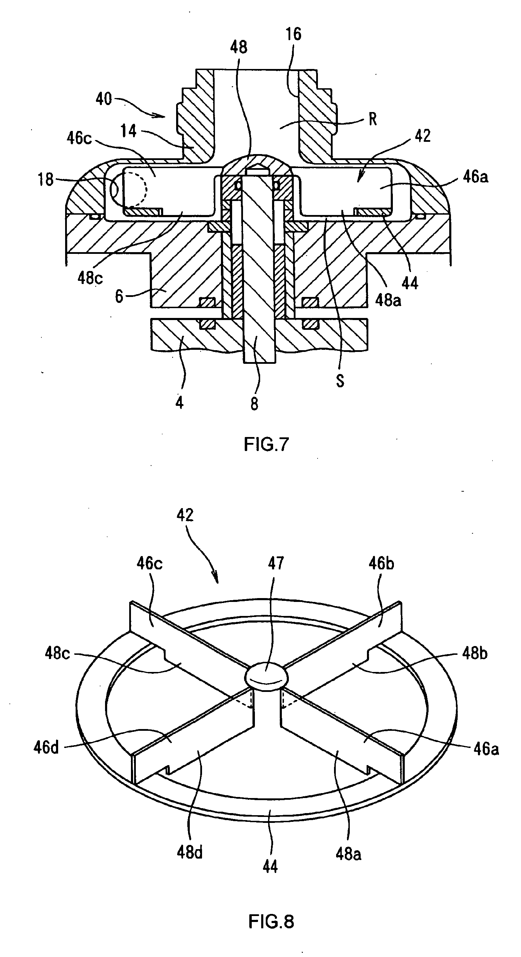

The following describes, with reference to FIGS. 7 to 9, a centrifugal pump according to a fourth embodiment that is used as a blood pump. It should be noted that this fourth embodiment corresponds to Claim 6. Components that are the same as in the construction shown in FIG. 1 have been given the same reference numerals and description of such has been omitted.

A centrifugal pump 40 of the present embodiment is a semi-open impeller-type centrifugal pump where a pump vane 42 is connected to an end part of the rotational shaft 8 that passes through the pump base part 6. The casing 14 that is connected to the end part of the pump base part 6 surrounds the pump vane 42 to form a fluid chamber R. The casing 14 is provided with the intake 16 that faces an upper part of a central position of the pump vane 42 and the outtake 18 that faces an outside in the radial direction of the pump vane 42.

As shown in FIG. 8, the pump vane 42 is composed of a first shroud 44 that is ring-shaped, a plurality of blades 46a to 46d that are disposed radially and whose outer edges are fixed onto the first shroud 44, and a rotation supporting member 47 that supports the inner edge parts of the blades 46a to 46d.

The plurality of blades 46a to 46d are integrally formed with current plates 48a to 48d that correspond to the stagnation preventing means of the present invention and point downwards towards the pump base part 6.

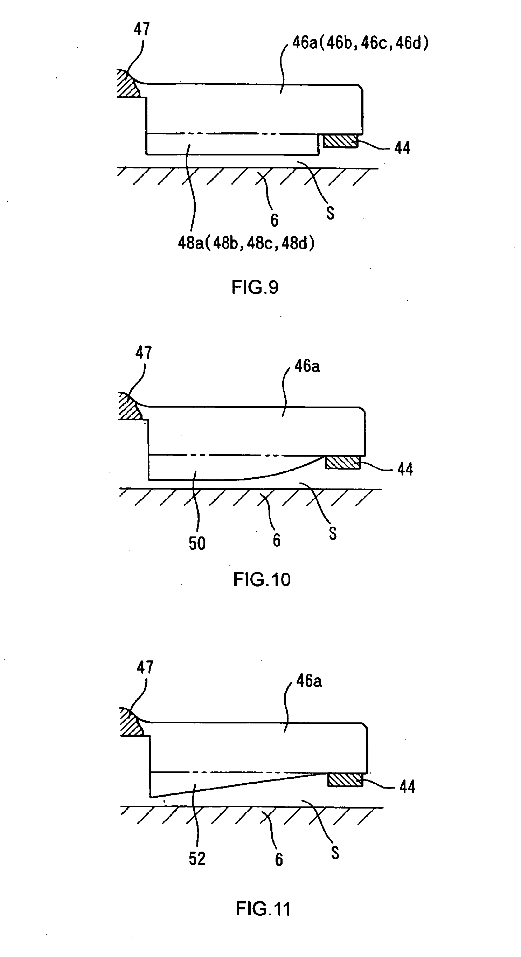

This is to say, as shown in FIG. 9, the current plates 48a to 48d are rectangular parts of the blades 46a to 46d in regions that extend downwards across the surfaces of the blades 46a to 46d and are positioned below the dot-dot-dash line. These current plates 48a to 48d project downwards beyond the lower surface of the first shroud 44.

In the centrifugal pump 40 of the above construction, the rotational shaft 8 is rotated by the driving unit 4, with the pump vanes 42 simultaneously rotating in a predetermined direction. A fluid flow in the centrifugal direction is generated in the blood in the pump vanes 42 that has entered the fluid chamber R from the intake 16, so that blood is pumped from the intake 16 to the outtake 18.

The current plates 48a to 48d, which are integrally formed with and rotate with the blades 46a to 46d, agitate the blood that stagnates in the gap S between the pump base part 6 and the lower surfaces of the pump vanes 42 so as to generate a centrifugal flow from the inner periphery to the outer periphery.

When some blood stagnates in the gap S, the current plates 48a to 48d agitate the blood in the gap S to generate a centrifugal flow from the inner periphery to the outer periphery, thereby stopping the blood from stagnating in the gap S and preventing a thrombus from forming in the blood pump. Since no blood stagnates, the centrifugal pump 40 does not suffer from a decrease in pump efficiency and so has superior pump performance.

The following describes, with reference to FIGS. 10 to 15, alternative embodiments for the current plates that extend downwards along the surfaces of the blades 46a to 46d.

The current plate 50 integrally formed at the bottom of the blade 46a (the other blades have the same construction) shown in FIG. 10 is formed so that the plate width is large on the inside (the rotation supporting member 47 side) and gradually decreases towards the outside (the first shroud 44 side) so that the lower edge of the blade 46a traces an arc. The current plate 52 that integrally formed at the bottom of the blade 46a shown in FIG. 11 is formed so that the plate width is large on the inside (the rotation supporting member 47 side) and gradually decreases towards the outside (the first shroud 44 side) so that the lower edge of the blade 46a traces a straight line that rises diagonally. These current plates 50, 52 shown in FIGS. 10 and 11 have an increased area on the inside and so can increase the centrifugal force, which means that blood that stagnates in the gap S can be efficiently pumped towards the outtake 18.

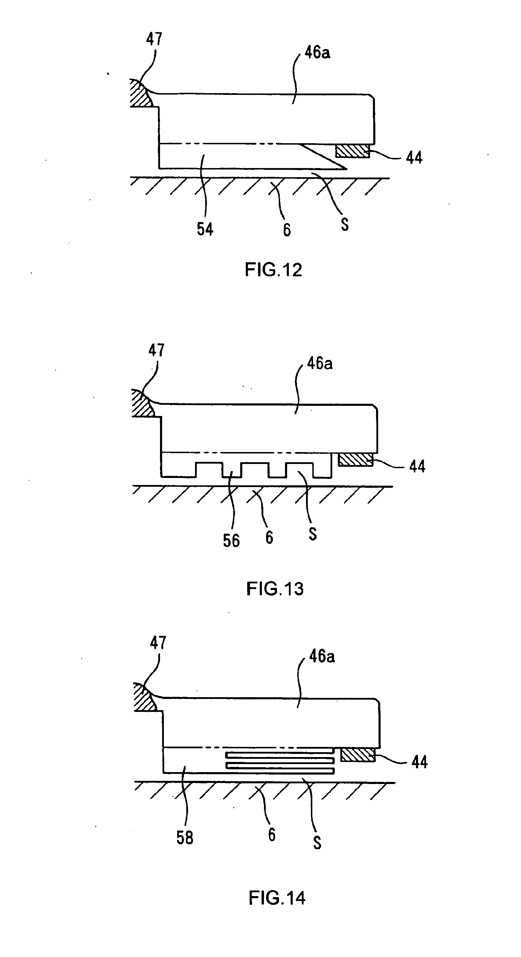

The current plate 54 shown in FIG. 12 is shaped with an increased area close to the pump base part 6, and by agitating the blood that stagnates in the gap S more aggressively, the centrifugal force is generated efficiently.

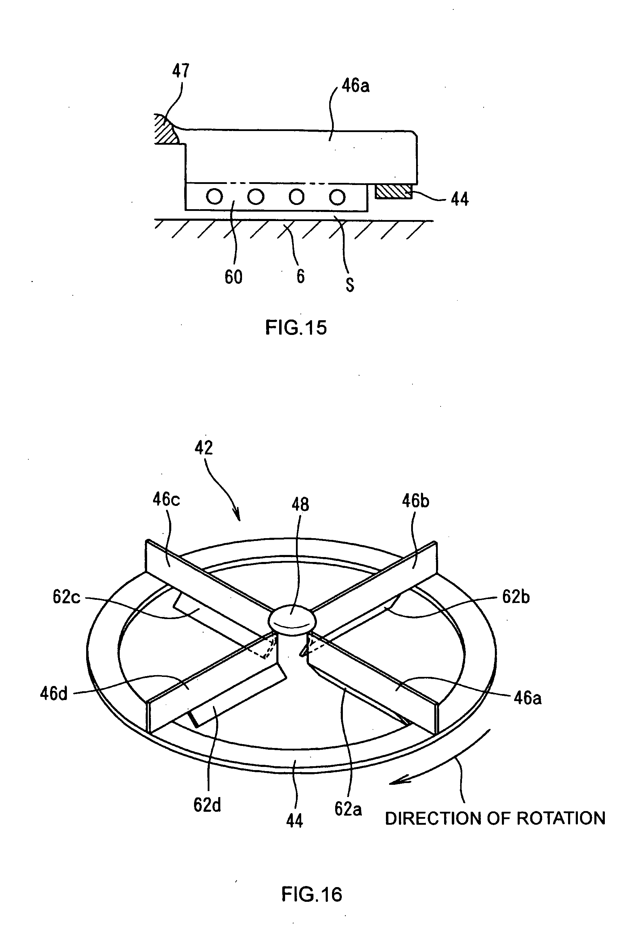

The current plate 56 shown in FIG. 13 has concave and convex parts that point towards the pump base part 6, while the current plate 58 shown in FIG. 14 is shaped like a comb and the current plate 60 shown in FIG. 15 has a plurality of through-holes. With the constructions shown in FIGS. 13 to 15, when the current plates 56, 58, 60 pass through the gap S, the convex/concave parts, the comb-shaped part or the through-holes generate small whirlpools in the blood, so that the blood in the gap S is constantly moving. This makes it difficult for blood to stagnate in the gap S.

FIG. 16 shows pump vanes 42 according to a fifth embodiment of the present invention that includes current plates that differ to the constructions shown in FIGS. 10 to 15.

The current plates 62a to 62d of the present embodiment are rectangular in shape and are bent so as to extend downwards diagonally with respect to the surfaces of the blades 46a to 46d. The direction in which the current plates 62a to 62d are bent is opposite to the direction in which the pump vanes 42 rotate.

The angle at which the current plates 62a to 62d are bent is set so that the vector of the axial flow of the blood matches the combined vector for the centrifugal flow vectors, which makes it possible to smoothly pump the blood that stagnates in the gap S to the outtake 18.

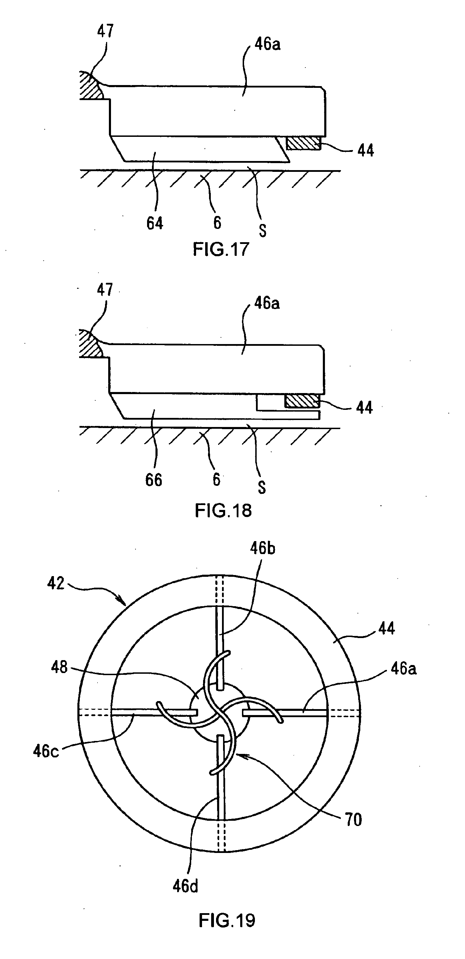

FIGS. 17 and 18 show current plates of a different construction to the current plates 62a to 62d that are shown in FIG. 16. The same effects as described above can be achieved with current plates 64 shaped as parallelograms as shown in FIG. 17 and with current plates 66 formed with parts that extend below the first shroud 44 as shown in FIG. 18. In particular, the current plates 66 shown in FIG. 18 can increase the speed of the centrifugal flow of blood that stagnates in the gap S.

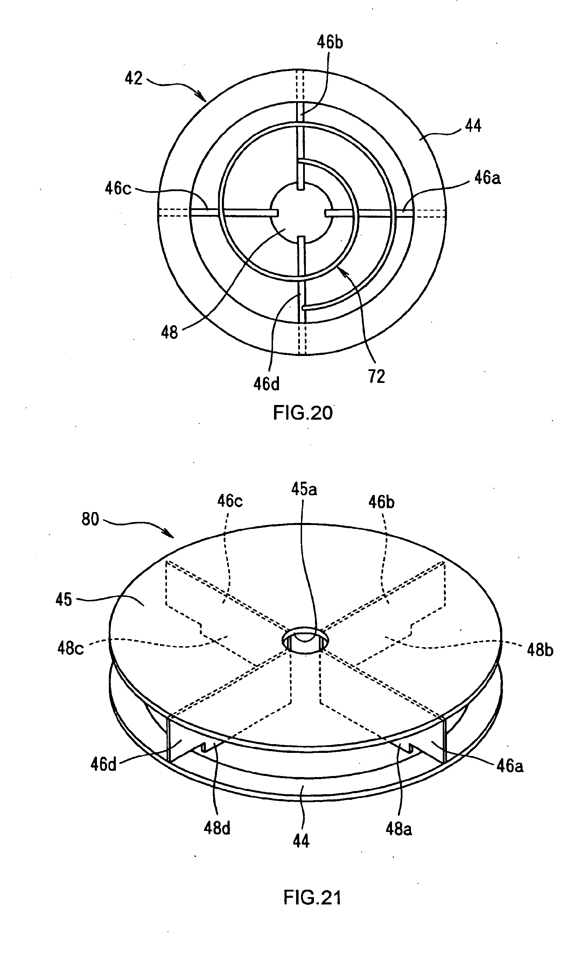

The following describes pump vanes according to sixth and seventh embodiments of the present invention, with reference to FIGS. 19 and 20.

FIG. 19 that illustrates the sixth embodiment shows the pump vane 42 from the rear side. The current plates are not integrally formed with the plurality of blades 46a to 46d and instead a current plate 70 that is in the approximate shape of a cross is connected so as span the intervals between the blades 46a to 46d. In FIG. 20 that illustrates the seventh embodiment, a current plate 72 in the form of a spiral is connected so as to span the intervals between the blades 46a to 46d.

With the pump vanes 42 of the sixth and seventh embodiments, the current plate 70 with the approximate shape of a cross and the spiral-shaped current plate 72 sufficiently agitate the blood in the gap S while generating a centrifugal flow from the inner periphery to the outer periphery. By doing so, as with the other embodiments, it is possible to provide a blood pump in which the stagnation of blood in the gap S is avoided and the formation of a thrombus is reliably prevented.

FIG. 21 shows an eighth embodiment of the present invention that is directed to a pump vane that composes a closed impeller-type centrifugal pump. It should be noted that this eighth embodiment corresponds to Claim 7. Also, components that are the same as in the construction of the centrifugal pump 40 shown in FIG. 7 have been given the same reference numerals and description of such has been omitted.

The pump vanes 80 of the present embodiment are constructed of the plurality of blades 46a to 46d that are disposed radially and are sandwiched between and fixed to the first shroud 44 and a disc-shaped second shroud 45 that are provided above and below the plurality of blades 46a to 46d. A fluid passage hole 45a is formed in the center of the second shroud 45. Current plates 48a to 48d are integrally formed with the blades 46a to 46d.

With a closed impeller-type centrifugal pump with the pump vanes 80 of the above construction, as with the fourth embodiment shown in FIGS. 7 and 8, the current plates 48a to 48d agitate the blood that stagnates in the gap S between the pump base part 6 and the lower surfaces of the pump vanes 42 and so generate a flow in the centrifugal direction from the inner periphery to the outer periphery. This stops blood from stagnating in the gap S and reliably prevents a thrombus from forming. In this closed impeller-type centrifugal pump also, there is no stagnation of blood, so that the pump efficiency does not decrease and the pump has superior pump performance.

By using the current plates shown in any of FIGS. 10 to 20, for example, in place of the current plates 48a to 48d that extend downwards from the surfaces of the plates 46a to 46d, the effects of the various embodiments described above can be achieved.

It should be noted that while the current plates extend below the blades in the semi-open-impeller-type centrifugal pumps or closed-impeller-type centrifugal pumps of the fourth to eighth embodiments described above, this is not a limitation for the present invention. As examples, the current plates may extend above the blades, below the first shroud 44, or above the second shroud 45.

It should be noted that while the effects of the above embodiments have been described for the case of a blood pump that is used as an artificial heart or the like, the present invention is not limited to such use. As one example, in the field of construction, by using the present invention in a conveyor pump for concrete where the hardening of the fluid causes quality problems, the stagnation of concrete inside the pump can be prevented, which stops the concrete hardening and improves the efficiency of the transportation of the concrete.

A centrifugal pump prevents a fluid from stagnating- and so stops decreases in pump efficiency. An open impeller-type centrifugal pump includes pump vanes 12 that are connected via a vane boss 10 to an end part of a rotational shaft 8, a casing 14 that is connected to an end part of the pump base part 6 and forms a fluid chamber R surrounding the pump vanes 12, and an intake 16 and an outtake 18 that are formed in the casing 14. The pump vanes 12 are composed of pump vanes 12a to 12c that are integrally formed with current plates 20a to 20c. The current plates 20a to 20c cause some of the fluid that enters via the intake 16 to flow downwards along the axial direction of the rotational shaft 8. The fluid that flows along the current plates causes a centrifugal flow from an inner periphery to an outer periphery in a gap between a pump base part and the lower surfaces of the pump vanes.

## Claims

1. A centrifugal pump including a rotational shaft that is disposed on a pump base part, at least one pump vane that is radially connected via a vane boss to an end part of the rotational shaft, a casing that is connected to the pump base part and forms a fluid chamber surrounding the at least one pump vane, a fluid intake that is provided at a predetermined position on the casing, and a fluid outtake that is provided at a predetermined position on the casing,
the centrifugal pump further comprising stagnation preventing means for generating, in a gap between the pump base part and lower surfaces of the at least one pump vane, a fluid flow in a centrifugal direction that is directed towards the fluid outtake.

2. A centrifugal pump according to Claim 1,
wherein the stagnation preventing means is at least one current plate that is provided on the vane boss, the at least one current plate causing some of a fluid that enters via the fluid intake to flow in an axial direction towards the pump base part, causing acceleration in the fluid flowing in the axial direction, and making the fluid flow towards the gap between the pump base part and the lower surfaces of the at least one pump vane.

3. A centrifugal pump according to Claim 2,
wherein each of the at least one current plate is integrally formed with base parts of the at least one pump vane and is connected to the vane boss.

4. A centrifugal pump according to Claim 2,
wherein each of the at least one current plate is a wing-shaped member that is connected to the vane boss at a different position to base parts of the at least one pump vane.

5. A centrifugal pump according to any of Claims 1 to 4,
wherein a plurality of current channels, which extend radially from the rotational shaft and are bent on the radial direction, are formed in at least one of a surface of the pump base part, the lower surfaces of the at least one pump vane, and lower surfaces of the at least one current plate.

6. A centrifugal pump including a pump vane in which a plurality of blades are disposed radially on a ring shaped first shroud, a casing that is connected to a pump base part and forms a fluid chamber surrounding the pump vane, a fluid intake that is provided at a predetermined position on the casing, and a fluid outtake that is provided at a predetermined position on the casing,
the centrifugal pump further comprising stagnation preventing means for generating, in a gap between a lower surface of the pump vane and the pump base part, a flow in a centrifugal direction that is directed towards the fluid outtake.

7. A centrifugal pump including a pump vane where a plurality of blades are disposed radially on a ring-shaped first shroud and a disc-shaped second shroud with a central fluid passage hole is disposed on the blades, a casing that is connected to a pump base part and forms a fluid chamber surrounding the pump vane, a fluid intake that is provided at a predetermined position on the casing, and a fluid outtake that is provided at a predetermined position on the casing,
the centrifugal pump further comprising stagnation preventing means for generating, in a gap between a lower surface of the pump vane and the pump base part, a flow in a centrifugal direction that is directed towards the fluid outtake.

8. A centrifugal pump according to any of Claims 6 and 7,
wherein the stagnation preventing means is at least one current plate that is disposed on the lower surface of the pump vane facing the pump base part, agitates fluid that stagnates in the gap between the lower surfaces of the pump vane and the pump base part, and generates the flow in the centrifugal direction towards the fluid outtake.

9. A centrifugal pump according to any of Claims 6 and 7,
wherein a plurality of current channels, which extend radially from a center of rotation of the pump vane and are bent on the radial direction, are formed in at least one of a lower surface of the first shroud, a lower surface of the pump vane, and lower surfaces of the at least one current plate.

10. A centrifugal pump according to any of Claims 1 to 9,
wherein the fluid is blood.
